(19) Europäisches Patentamt
European Patent Office
Office européen des brevets

(11) **EP 2 837 350 B1**

(12) **EUROPEAN PATENT SPECIFICATION**

(45) Date of publication and mention
of the grant of the patent:
**21.09.2016 Bulletin 2016/38**

(51) Int Cl.:
*A61B 18/14* (2006.01)       *A61B 5/00* (2006.01)

(21) Application number: **14184482.9**

(22) Date of filing: **16.12.2013**

(54) **Device for assessment of optical lesion**

Vorrichtung zur optischen Läsionsbeurteilung

dispositif pour évaluation de lésion optique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2012 US 201213716517**

(43) Date of publication of application:
**18.02.2015 Bulletin 2015/08**

(62) Document number(s) of the earlier application(s) in accordance with Art. 76 EPC:
**13197493.3 / 2 742 892**

(73) Proprietor: **Biosense Webster (Israel) Ltd.**
**20692 Yokneam (IL)**

(72) Inventors:
• **Govari, Assaf**
**34400 Haifa (IL)**
• **Beeckler, Christopher Thomas**
**Brea, CA California 92821 (US)**
• **Papaioannou, Athanassios**
**Los Angeles, CA California 90066 (US)**
• **Gliner, Vadim**
**33851 Haifa (IL)**

(74) Representative: **Carpmaels & Ransford LLP**
**One Southampton Row**
**London WC1B 5HA (GB)**

(56) References cited:
WO-A1-2012/049621       US-A1- 2008 119 694
US-A1- 2009 005 773       US-A1- 2010 241 100

EP 2 837 350 B1

**Description**

**FIELD OF THE INVENTION**

The present disclosure relates generally to invasive medical devices and procedures, and particularly to assessment of the condition of tissue treated in such procedures.

**BACKGROUND**

Minimally-invasive intracardiac ablation is the treatment of choice for various types of arrhythmias. To perform such treatment, the physician typically inserts a catheter through the vascular system into the heart, brings the distal end of the catheter into contact with myocardial tissue in areas of abnormal electrical activity, and then energizes one or more electrodes at or near the distal end in order to create tissue necrosis.

It is often difficult to determine the proper dosage of energy that should be applied in an ablation procedure in order to achieve the desired result. When the dosage is insufficient, the non-conducting lesion will not extend deeply enough through the heart wall to disrupt the abnormal conduction, so that arrhythmia may persist or return after the procedure is completed. On the other hand, excessive dosage may cause dangerous damage to the tissue at and around the ablation site. The proper dosage is known to vary from case to case depending on various factors, such as catheter geometry, thickness of the heart wall, quality of the electrical contact between the catheter electrode and the heart wall, and blood flow in the vicinity of the ablation site.

In order to improve the precision and consistency of ablation procedures, attempts have been made to predict and control the ablation based on measurement of physiological parameters of relevance. Some methods of this sort are described, for example, in U.S. Patent 7,306, 593 and in the patent application US 2009/0005773 A1.

As another example, U.S. Patent 7,918,850 describes monitoring of intracardiac ablation progress in near-real-time by evaluating capture of a pacing signal.

Optical methods of intracardiac lesion assessment are also known in the art. For example, U.S. patent 7,662,152 describes a catheter comprising a catheter body and a tip electrode adapted for ablating tissue. The catheter further includes a plurality of optical waveguides adapted to transmit optical energy to and from the tip electrode. A distal portion of each waveguide extends through a hollow distal portion of the tip electrode and terminates in openings formed in the shell. Lesion assessments are accomplished by measuring the light intensity at one or more wavelengths that is recaptured at the catheter tip resulting from the light radiated from the catheter tip onto ablated tissue. U.S. Patent 8,123,745, describes an ablation catheter with an optically transparent, electrically conductive tip for similar purposes.

**SUMMARY**

The invention is defined in the appended claim. Methods mentioned hereinafter do not form part of the invention.

Embodiments of the present invention that are described hereinbelow provide improved methods and devices for measuring optical properties of tissue within the body. Such methods and devices may be used effectively in optical lesion assessment.

There is therefore provided, in accordance with an embodiment of the present invention, medical apparatus, including a probe, having a distal segment configured for insertion into a body of a patient. The probe includes at least one optical sensing unit, which is disposed along the distal segment and includes first and second radiation sources, configured to emit optical radiation in different, respective, first and second wavelength bands toward tissue in the body in proximity to the distal segment. An optical sensor is configured to receive the optical radiation in the first and second wavelength bands that is scattered from the tissue and to output first and second electrical signals responsively to an intensity of the received optical radiation.

In some embodiments, the first wavelength band is an infrared band, and the second wavelength band is a visible light band. For example, the first wavelength band may have a peak intensity between 860 and 880 nm, while the second wavelength band has a peak intensity between 710 and 730 nm.

Typically, the apparatus includes a control unit, which is coupled to make a comparison of the first and second signals, and to output an indication of a condition of the tissue responsively to the comparison. The indication may be based on a ratio of the first and second signals.

In a disclosed embodiment, the distal segment of the probe includes an ablation element, which is configured to ablate the tissue, and the indication provides an assessment of a lesion formed in the tissue by the ablation element. The ablation element may include an electrode, which is configured to be brought into contact with the tissue and to ablate the tissue by applying radio-frequency energy to the tissue, wherein the control unit is configured to provide the assessment of the lesion as the lesion is formed during application of the radio-frequency energy. In one embodiment, the distal segment of the probe is configured to be brought into contact with and to ablate endocardial tissue within a heart of the patient.

In some embodiments, the first and second radiation sources include light-emitting diodes, which are embedded in the distal segment.

Optionally, the at least one optical sensing unit includes multiple optical sensing units, which are disposed at different, respective locations along the distal segment. In one embodiment, the multiple optical sensing units include at least first and second optical sensing units, which are spaced apart along the distal segment, and the apparatus includes a control unit, which is configured to communicate with the first and second optical sensing units so as to measure the signals output by the optical sensor in the first optical sensing unit responsively to the radiation emitted, in alternation, by the radiation sources in each of the first and second optical sensing units.

In another embodiment, the distal segment includes a cap including an outer wall, which is perforated by one or more apertures, and an inner wall, which is contained inside the outer wall and on which the at least one optical sensing unit is mounted so as to emit and receive the optical radiation toward and from the tissue via the apertures in the outer wall. The outer wall may include a conductive material, which is configured to be brought into contact with the tissue and to apply electrical energy to the tissue so as to ablate the tissue, while an irrigation fluid flows through a cavity between the inner and outer walls and exits the cavity through the one or more apertures.

The apparatus according to the present invention may be used in a method for tissue assessment, which includes inserting a distal segment of a probe into a body of a patient. First and second radiation sources, disposed along the distal segment, are actuated to emit optical radiation in different, respective, first and second wavelength bands toward tissue in the body in proximity to the distal segment. An optical sensor disposed along the distal segment receives the optical radiation in the first and second wavelength bands that is scattered from the tissue. First and second electrical signals, which are output by the optical sensor responsively to an intensity of the received optical radiation in the first and second wavelength bands, respectively, are processed in order to assess a condition of the tissue.

A further method for tissue assessment includes applying radio-frequency (RF) electrical energy so as to form a lesion in a region of a tissue inside a body of a patient. A first scattering intensity of the region to infrared radiation and a second scattering intensity of the region to red light are measured while applying the RF electrical energy. Formation of the lesion is assessed by comparing the first scattering intensity to the second scattering intensity.

The present invention will be more fully understood from the following detailed description of the embodiments thereof, taken together with the drawings in which:

## BRIEF DESCRIPTION OF THE DRAWINGS

Fig. 1 is a schematic pictorial illustration of a system for intracardiac ablation, in accordance with an embodiment of the present invention;
Fig. 2A is a schematic pictorial illustration of a distal segment of an ablation and sensing catheter, in accordance with an embodiment of the present invention;
Fig. 2B is a schematic pictorial illustration of a distal segment of an ablation and sensing catheter, in accordance with another embodiment of the present invention;
Fig. 3 is a plot of a spectral reflectance ratio measured by a catheter during an ablation procedure, in accordance with an embodiment of the present invention; and
Fig. 4 is a schematic, sectional illustration of a distal segment of an ablation and sensing catheter, in accordance with another embodiment of the present invention.

## DETAILED DESCRIPTION OF EMBODIMENTS

Embodiments of the present invention that are described hereinbelow provide devices and methods that can be used to evaluate tissue condition within the body quickly and accurately, by comparing the scattering intensity from tissue at different wavelengths. Specifically, the inventors have found that the relation between infrared and visible light scattering from tissue inside the body, and particularly heart tissue, changes distinctly as the tissue is ablated, with a sharp increase in the ratio of infrared to visible light scattered. A suitable optical sensing unit in the ablation probe (such as in a cardiac ablation catheter) can thus be used to assess lesion formation in real time during an ablation procedure. The term "scattering" is used in the present disclosure and in the claims in its broad, conventional sense, to include generally both reflected and transmitted radiation that reaches an optical sensor via the tissue in question, or in other words, both backward and forward scattering.

In the disclosed embodiments, at least one optical sensing unit is disposed along a distal segment of a probe, which is inserted into the body of a patient. This sensing unit comprises (at least) two radiation sources, which emit optical radiation in different, respective wavelength bands toward the tissue in proximity to the distal segment within the body. An optical sensor in the sensing unit receives the optical radiation that is scattered from the tissue in the different wavelength bands and outputs electrical signals in response to the radiation intensity. The term "optical radiation," as used in the present description and in the claims, includes visible, infrared and ultraviolet radiation. Typically (although not necessarily), the radiation sources are light-emitting diodes (LEDs), one of which emits infrared radiation, and the other visible light, in different, respective timeslots. The radiation sources and the sensor in the sensing unit may be positioned in close proximity to one another, or they may alternatively be spread apart at different locations along the distal segment.

A control unit, coupled to the probe, compares the signals that are output by the sensor in response to the intensity of the scattered radiation received in the different wavelength bands and generates an indication of the condition of the tissue based on this comparison. This indication may typically comprise an assessment of lesion formation during tissue ablation performed by the probe. The indication may be based, for example, on the ratio of infrared to visible light scattering, which the inventors have found to increase sharply as tissue is ablated.

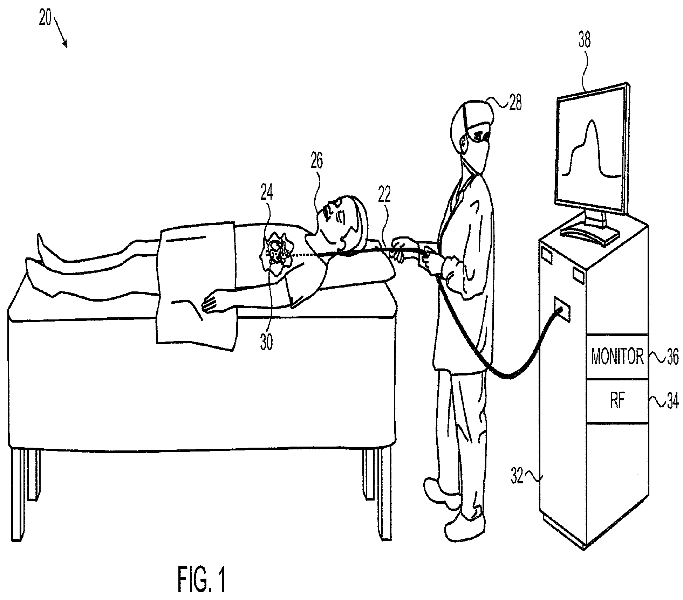

Fig. 1 is a schematic pictorial illustration of a system 20 for cardiac ablation treatment, in accordance with an embodiment of the present invention. An operator 28 (such as an interventional cardiologist) inserts an invasive probe, such as a catheter 22, via the vascular system of a patient 26 into a chamber of the patient's heart 24. For example, to treat atrial fibrillation, the operator may advance the catheter into the left atrium and bring a distal segment 30 of the catheter into contact with myocardial tissue that is to be ablated.

Catheter 22 is connected at its proximal end to a console 32, which serves as a control unit in applying and monitoring the desired treatment, under the control of operator 28. Console 32 comprises a radio-frequency (RF) energy generator 34, which supplies electrical power via catheter 22 to distal segment 30 in order to ablate the target tissue. Monitoring circuitry 36 provides an indication of the condition of the tissue in proximity to distal segment 30 by processing the signals output by one or more optical sensing units along the distal segment, as described below, and may display this indication on a display screen 38. Typically, an irrigation pump (not shown) supplies a cooling fluid, such as saline solution, through catheter 22 to irrigate the tissue under treatment by distal segment 30. On the basis of information provided by monitoring circuitry 36, console 32 may control the power applied by RF energy generator 34 and/or the flow of fluid provided by the pump, either automatically or in response to inputs by operator 28.

System 20 may be based, for example, on the CARTO system offered by Biosense Webster Inc. (Diamond Bar, California), which provides extensive facilities to support navigation and control of catheter 22. These system facilities, however, including details of the monitoring and control functions of console 32 generally (other than the optical sensing functions described herein), are beyond the scope of the present patent application.

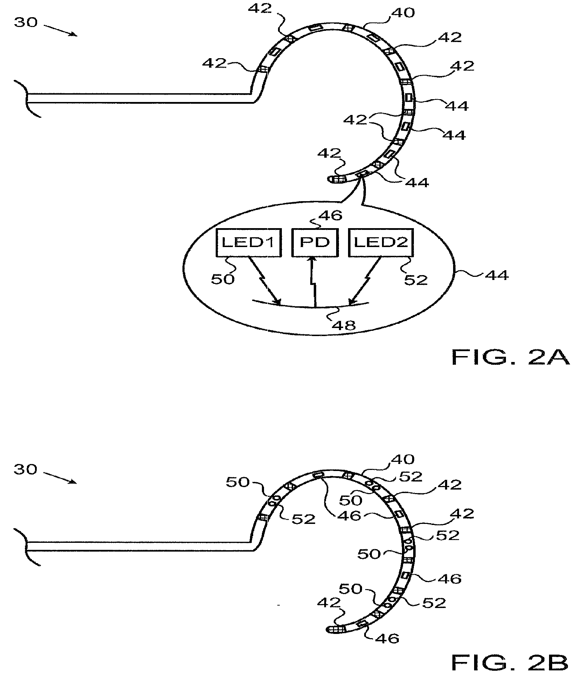

Fig. 2A is a schematic, pictorial illustration of distal segment 30 of catheter 22, in accordance with an embodiment of the present invention. In this example, the distal segment is shown as comprising an arcuate portion 40, having the form of a "lasso." This sort of catheter form is commonly used in creating annular lesions, around the ostia of the pulmonary veins, for example, for treatment of atrial fibrillation. For this purpose, distal segment 30 is brought into contact with endocardial tissue against all, or at least a part of, the length of arcuate portion 40. Ablation elements, in the form of electrodes 42, are disposed along the length of distal segment 30 and are actuated with RF energy by generator 34 to ablate the tissue with which they are in contact. Alternatively, distal segment 30 may comprise other sorts of ablation elements, such as high-power ultrasonic transducers or cryo-ablation elements, as are known in the art.

Optical sensing units 44 are disposed along distal segment 30, at locations that are interleaved between electrodes 42. Each such unit 44, as shown in the inset in Fig. 2A, comprises two radiation sources 50 and 52, which direct optical radiation in different, respective wavelength bands toward myocardial tissue 48 in proximity to the sensing unit. The inventors have found that for assessment of lesion formation, radiation source 50 may advantageously emit infrared radiation while radiation source 52 emits visible light, such as red light. The wavelength bands may have respective peak intensities, for example, between 860 and 880 nm and between 710 and 730 nm. In one embodiment, sources 50 and 52 comprise LEDs with center emission wavelengths at approximately 870 and 720 nm, respectively.

Alternatively, other wavelength combinations may be useful for assessment of ablation and other indications such as assessing contact between the catheter and body tissue. For example, choosing a wavelength in the visible spectrum that is highly absorbed by hemoglobin in the blood may be useful, because in the absence of good tissue contact, the scattering intensity at that wavelength will be close to zero. Upon contact with the tissue, the displacement of the blood results in reduced absorption, thus giving an increased signal that is indicative of tissue contact. Furthermore, upon initiation of ablation, the loss of oxygenated blood (in addition to other changes in the tissue optical properties) and consequent reduction in absorption can provide further information on the ablation process.

Each sensing unit 44 also comprises an optical sensor 46, such as a photodiode or other suitable radiation-sensing element, which receives the optical radiation that is reflected from tissue 48 and outputs electrical signals in response to the intensity of the received radiation. Typically, radiation sources 50 and 52 are time-multiplexed, so that the respective signals output by sensor 46 due to the reflected radiation at the two wavelengths can be clearly distinguished. Optionally, sensing unit 44 may comprise three or more radiation sources, each with its own emission wavelength, so that sensor 46 may measure tissue reflectance with finer wavelength resolution and/or over a wider range of wavelengths. Radiation sources 50, 52 and sensor 46 are typically embedded in distal segment 30 within a suitable transparent, sealed envelope, such as a transparent biocompatible plastic.

Monitoring circuitry 36 receives the signals that are output by sensor 46 and compares the signal levels due to scattering of the radiation at the different wavelengths of sources 50 and 52 in order to derive an indication of the level of ablation of tissue 48. In this manner, console 32 is able to assess the lesion being formed during the ablation process and may present this assessment on display 38. Typically, as illustrated below, this comparison of the signal levels is based on the ratio of signals due to scattering at the different wavelengths of sources 50 and 52, but alternatively or additionally, other mathematical relations may be used in analyzing the signals.

In the embodiment shown in Fig. 2A, multiple optical sensing units 44 are disposed at different, respective locations, spaced apart along arcuate portion 40 of distal segment 30. Alternatively, catheter 22 may comprise only a single optical sensing unit of this sort, but the use of multiple sensing units enables console 32 to assess lesion formation over a wider region. To broaden the region of assessment still further to the areas between the different optical sensing units along the length of the distal segment, monitoring circuitry 36 may control radiation sources 50, 52 in neighboring sensing units 44 to operate in alternation, so that sensor 46 in one of the optical sensing units can measure scattering of radiation, including both transmission and reflection, from the radiation sources in the neighboring sensing unit(s). This scattering takes place in tissue 48 in between the neighboring sensing units 44 and thus give an indication of lesion formation in these intermediate areas.

Fig. 2B is a schematic, pictorial illustration of distal segment 30 of catheter 22, in accordance with an alternative embodiment of the present invention. In this embodiment, sources 50, 52 and sensors 46 are spaced apart at different locations along the length of arcuate portion 40. Any combination of a pair of sources 50, 52 and a sensor 46 may be treated as an optical sensing unit in this configuration, in order to sample the scattering from the area of tissue between the selected sources and sensor.

Although Figs. 2A and 2B show particular numbers of optical sensing units 44 disposed in certain locations and configurations along distal segment 30, in alternative embodiments substantially any number of such optical sensing units may be used, and possibly only a single optical sensing unit. Moreover, although Figs. 2A and 2B shows a lasso catheter, in other embodiments optical sensing units of this sort may be fitted to other types of catheters and other invasive probes having any suitable configuration, for use not only in the heart but also in other body organs and regions.

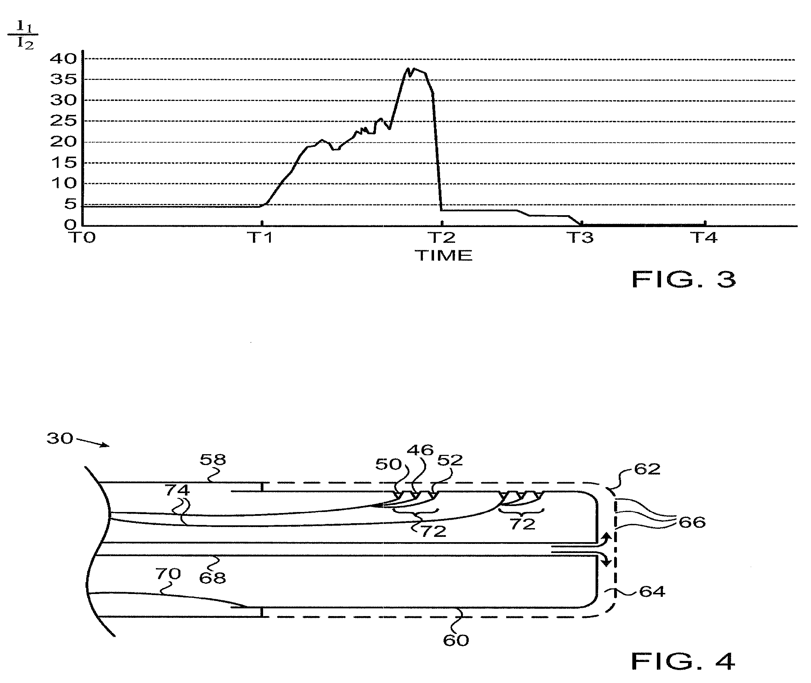

Fig. 3 is a plot of the spectral ratio of the received signals measured by a catheter system using optical sensing unit 44, as a function of time during an ablation procedure, in accordance with an embodiment of the present invention. Radiation sources 50 and 52 in this example are LEDs emitting in wavelength bands having peak intensities at 870 nm and 720 nm, respectively. The vertical axis shows the ratio $\frac{I(870)}{I(720)}$ (marked in the figure as I1/I2) of the intensity scattered from tissue 48 at the two different wavelengths, based on the signals output by sensor 46. This figure illustrates the effectiveness of this intensity ratio in assessing tissue ablation.

During the initial time period before ablation begins, from T0 to T1, the baseline scattering intensity ratio for unablated tissue is approximately 5:1. RF energy is applied to the catheter electrode starting at time T1. As a lesion is formed by ablation, the ratio gradually increases to approximately 40:1. At time T2, the RF energy is turned off, and the catheter is moved so that sensing unit 44 views another unablated tissue region, and the ratio returns to the previous value of approximately 5:1. When the catheter is withdrawn from the tissue, at time T3, sensor 44 receives scattered radiation only from blood cells in the heart chamber, and the intensity ratio drops to nearly zero.

Fig. 4 is a schematic, sectional illustration of distal segment 30 of an ablation and sensing catheter, in accordance with another embodiment of the present invention. In this embodiment, a cap, attached to the distal end of an insertion tube 58 of the catheter, comprises an outer wall 62, which is perforated by apertures 66, and an inner wall 60, which is contained inside the outer wall. A lumen 68 supplies irrigation fluid to a cavity 64 that is formed between outer wall 62 and inner wall 60, and the irrigation fluid exits this cavity through apertures 66. Typically, walls 60 and 62 comprise thin shells of metallic material, which are held apart by small metallic spacers (not shown), around which the fluid is able to flow within cavity 64. A conductor 70 supplies RF electrical energy from console 32 to the cap, which serves as an electrode to ablate tissue with which outer wall 62 is in contact.

Optical sensing units 72 are mounted on inner wall 60 so that sources 50, 52 emit optical radiation through apertures 66 toward tissue in proximity to the cap, and sensors 46 receive reflected radiation via the apertures. Sources 50, 52 and sensor 46 may be inset in indentations within the inner wall, as shown in the figure. This inset configuration may be useful in guiding the radiation emitted from the sources outward toward the tissue, as well as limiting the angular extent of the reflected radiation that is observed by the sensor.

Although a number of particular optical sensing unit configurations are shown and described above, alternative configurations that may be used for similar purposes will be apparent to those skilled in the art after reading the above description and are considered to be within the scope of the present invention. It will thus be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

Aspects of the invention not yet claimed:

Aspect 1. A method for tissue assessment, comprising:

inserting a distal segment of a probe into a body of a patient;

actuating first and second radiation sources, disposed along the distal segment, to emit optical radiation in different, respective, first and second wavelength bands toward tissue in the body in proximity to the distal segment;

receiving, at an optical sensor disposed along the distal segment, the optical radiation in the first and second wavelength bands that is scattered from the tissue; and

processing first and second electrical signals, which are output by the optical sensor responsively to an intensity of the received optical radiation in the first and second wavelength bands, respectively, in order to assess a condition of the tissue.

Aspect 2. The method according to aspect 1, wherein the first wavelength band is an infrared band, and the second wavelength band is a visible light band.

Aspect 3. The method according to aspect 1, and comprising ablating the tissue using the probe, wherein processing first and second electrical signals comprises assessing formation of a lesion in the tissue.

Aspect 4. The method according to aspect 3, wherein ablating the tissue comprises applying radio-frequency energy to the tissue, and wherein the formation of the lesion is assessed as the lesion is formed during application of the radio-frequency energy.

Aspect 5. The method according to aspect 3, wherein inserting the distal segment comprises inserting a catheter into a heart of the patient so as to ablate endocardial tissue in the heart.

Aspect 6. The method according to aspect 1, wherein the first and second radiation sources comprise light-emitting diodes, which are embedded in the distal segment.

Aspect 7. The method according to aspect 1, wherein actuating the first and second radiation sources and receiving the optical radiation comprise emitting and receiving the optical radiation at multiple optical sensing units, which are disposed at different, respective locations along the distal segment.

Aspect 8. The method according to aspect 7, wherein the multiple optical sensing units comprise at least first and second optical sensing units, which are spaced apart along the distal segment, and wherein the method comprises measuring the signals output by the optical sensor in the first optical sensing unit responsively to the radiation emitted, in alternation, by the radiation sources in each of the first and second optical sensing units.

Aspect 9. The method according to aspect 1, wherein the distal segment comprises a cap comprising an outer wall, which is perforated by one or more apertures, and an inner wall, which is contained inside the outer wall and on which the first and second radiation sources and the optical sensor are mounted so as to emit and receive the optical radiation toward and from the tissue via the apertures in the outer wall.

Aspect 10. A method for tissue assessment, comprising:

applying radio-frequency (RF) electrical energy so as to form a lesion in a region of a tissue inside a body of a patient;

measuring a first scattering intensity of the region to infrared radiation and a second scattering intensity of the region to red light while applying the RF electrical energy; and

assessing formation of the lesion by comparing the first scattering intensity to the second scattering intensity.

Aspect 11. The method according to aspect 10, wherein the infrared radiation has a peak intensity between 860 and 880 nm, and the red light has a peak intensity between 710 and 730 nm.

Aspect 12. The method according to aspect 10, wherein comparing the first scattering intensity to the second scattering intensity comprises computing a ratio of signals corresponding to the first and second scattering intensities.

Aspect 13. The method according to aspect 10, wherein applying the RF electrical radiation comprises ablating endocardial tissue using a distal segment of a catheter that is inserted into a heart of the patient, and wherein the first and second scattering intensities are measured using an optical sensing unit on the distal segment.

Aspect 14. Medical apparatus, comprising a probe, having a distal segment configured for insertion into a body of a patient, the probe comprising at least one optical sensing unit, which is disposed along the distal segment and comprises:

first and second radiation sources, configured to emit optical radiation in different, respective, first and second wavelength bands toward tissue in the body in proximity to the distal segment; and

an optical sensor, which is configured to receive the optical radiation in the first and second wavelength bands that is scattered from the tissue and to output first and second electrical signals responsively to an intensity of the received optical radiation.

Aspect 15. The apparatus according to aspect 14, wherein the first wavelength band is an infrared band, and the second wavelength band is a visible light band.

Aspect 16. The apparatus according to aspect 15, wherein the first wavelength band has a peak intensity between 860 and 880 nm, and the second wavelength band has a peak intensity between 710 and 730 nm.

Aspect 17. The apparatus according to aspect 15, and comprising a control unit, which is coupled to make a comparison of the first and second signals, and to output an indication of a condition of the tissue responsively to the comparison.

Aspect 18. The apparatus according to aspect 17, wherein the distal segment of the probe comprises an ablation element, which is configured to ablate the tissue, and wherein the indication provides an assessment of a lesion formed in the tissue by the ablation element.

Aspect 19. The apparatus according to aspect 18, wherein the ablation element comprises an electrode, which is configured to be brought into contact with the tissue and to ablate the tissue by applying radio-frequency energy to the tissue, and wherein the control unit is configured to provide the assessment of the lesion as the lesion is formed during application of the radio-frequency energy.

Aspect 20. The apparatus according to aspect 14, wherein the distal segment of the probe is configured to be brought into contact with and to ablate endocardial tissue within a heart of the patient.

Aspect 21. The apparatus according to aspect 17, wherein the indication is based on a ratio of the first and second signals.

Aspect 22. The apparatus according to aspect 14, wherein the first and second radiation sources comprise light-emitting diodes, which are embedded in the distal segment.

Aspect 23. The apparatus according to aspect 14, wherein the at least one optical sensing unit comprises multiple optical sensing units, which are disposed at different, respective locations along the distal segment.

Aspect 24. The apparatus according to aspect 23, wherein the multiple optical sensing units comprise at least first and second optical sensing units, which are spaced apart along the distal segment, and

wherein the apparatus comprises a control unit, which is configured to communicate with the first and second optical sensing units so as to measure the signals output by the optical sensor in the first optical sensing unit responsively to the radiation emitted, in alternation, by the radiation sources in each of the first and second optical sensing units.

Aspect 25. The apparatus according to aspect 14, wherein the distal segment comprises a cap comprising an outer wall, which is perforated by one or more apertures, and an inner wall, which is contained inside the outer wall and on which the at least one optical sensing unit is mounted so as to emit and receive the optical radiation toward and from the tissue via the apertures in the outer wall.

Aspect 26. The apparatus according to aspect 25, wherein the outer wall comprises a conductive material, which is configured to be brought into contact with the tissue and to apply electrical energy to the tissue so as to ablate the tissue, while an irrigation fluid flows through a cavity between the inner and outer walls and exits the cavity through the one or more apertures.

**Claims**

1. Medical apparatus, comprising a probe, having a distal segment configured for insertion into a body of a patient, the probe comprising at least one optical sensing unit, which is disposed along the distal segment and comprises:

   first and second radiation sources, configured to emit optical radiation in different, respective, first and second wavelength bands toward tissue in the body in proximity to the distal segment; and
   an optical sensor, which is configured to receive the optical radiation in the first and second wavelength bands that is scattered from the tissue and to output first and second electrical signals responsively to an intensity of the received optical radiation,
   wherein the distal segment comprises a cap comprising an outer wall, which is perforated by one or more apertures, and an inner wall, which is contained inside the outer wall and on which the at least one optical sensing unit is mounted so as to emit and receive the optical radiation toward and from the tissue via the one or more apertures in the outer wall,

and wherein the outer wall comprises a metallic conductive material, which is configured to be brought into contact with the tissue and to apply electrical energy to the tissue so as to ablate the tissue, while an irrigation fluid flows through a cavity between the inner and outer walls and exits the cavity through the one or more apertures.

## Patentansprüche

1. Medizinische Vorrichtung, umfassend eine Sonde mit einem distalen Segment, das zum Einführen in den Körper eines Patienten konfiguriert ist, wobei die Sonde mindestens eine optische Erfassungseinheit umfasst, die entlang dem distalen Segment angeordnet ist und Folgendes umfasst:

eine erste und eine zweite Strahlungsquelle, die dazu konfiguriert sind, optische Strahlung in einem ersten bzw. zweiten Wellenlängenbereich, die jeweils verschieden sind, zu Gewebe im Körper in der Nähe des distalen Segments hin zu emittieren, und

einen optischen Sensor, der dazu konfiguriert ist, die optische Strahlung im ersten und zweiten Wellenlängenbereich, die vom Gewebe gestreut wird, zu empfangen und als Reaktion auf eine Intensität der empfangenen optischen Strahlung ein erstes und ein zweites elektrisches Signal auszugeben,

wobei das distale Segment eine Kappe umfasst, die eine Außenwand, die mit einer oder mehreren Öffnungen perforiert ist, und eine Innenwand umfasst, die innerhalb der Außenwand enthalten ist und an der die mindestens eine optische Erfassungseinheit montiert ist, um die optische Strahlung über die eine oder die mehreren Öffnungen in der Außenwand an das Gewebe zu emittieren und sie vom Gewebe zu empfangen,

und wobei die Außenwand ein metallisches leitendes Material umfasst, das dazu konfiguriert ist, mit dem Gewebe in Kontakt gebracht zu werden und das Gewebe mit elektrischer Energie zu beaufschlagen, um das Gewebe abzutragen, während ein Irrigationsfluid durch einen Hohlraum zwischen der Innen- und der Außenwand fließt und durch die eine oder die mehreren Öffnungen aus dem Hohlraum austritt.

## Revendications

1. Appareil médical, comprenant une sonde, comportant un segment distal configuré en vue de son insertion dans un corps d'un patient, la sonde comprenant au moins une unité de détection optique, qui est disposée le long du segment distal et comprend :

des première et seconde sources de rayonnement, configurées pour émettre un rayonnement optique dans des première et seconde bandes de longueur d'onde respectives, différentes, en direction d'un tissu dans le corps à proximité du segment distal ; et un capteur optique, qui est configuré pour recevoir le rayonnement optique dans les première et seconde bandes de longueur d'onde qui est diffusé par le tissu et pour produire en sortie des premier et second signaux électriques en réaction à une intensité du rayonnement optique reçu,

dans lequel le segment distal comprend un capuchon comprenant une paroi extérieure, qui est perforée par une ou plusieurs ouvertures, et une paroi intérieure, qui est contenue à l'intérieur de la paroi extérieure et sur laquelle la ou les unités de détection optiques sont montées de façon à émettre et recevoir le rayonnement optique en direction et en provenance du tissu par la ou les ouvertures dans la paroi extérieure, et dans lequel la paroi extérieure comprend un matériau métallique conducteur, qui est configuré pour être amené en contact avec le tissu et pour appliquer une énergie électrique au tissu de façon à effectuer l'ablation du tissu, tandis qu'un fluide d'irrigation s'écoule à travers une cavité entre les parois intérieure et extérieure et sort de la cavité par la ou les ouvertures.

FIG. 1

FIG. 2B

FIG. 2A

FIG. 4

FIG. 3

**EP 2 837 350 B1**

**Patent documents cited in the description**

- US 7306593 A
- US 20090005773 A1
- US 7918850 A
- US 7662152 A
- US 8123745 B